(19) Europäisches Patentamt
European Patent Office
Office européen des brevets

(11) EP 2 808 017 B1

(12) **EUROPEAN PATENT SPECIFICATION**

(45) Date of publication and mention of the grant of the patent:
**19.12.2018 Bulletin 2018/51**

(51) Int Cl.:
*A61K 31/37* (2006.01) *A61K 36/39* (2006.01)
*A61P 1/04* (2006.01)

(21) Application number: **13169307.9**

(22) Date of filing: **27.05.2013**

(54) **EXTRACT AND ISOLATED COMPOUNDS FROM CONVOLVULUS AUSTROAEGYPTIACUS FOR USE AS ANTI-ULCER AGENT AND/OR ANTI-HELIOBACTER PYLORI AGENT**

EXTRAKT- UND ISOLIERTE VERBINDUNGEN AUS CONVOLVULUS AUSTROAEGYPTIACUS ZUR VERWENDUNG GEGEN MAGENGESCHWÜRE UND/ODER MITTEL GEGEN HELIOBACTER PYLORI

EXTRAIT ET COMPOSÉS ISOLÉS À PARTIR DE CONVOLVULUS AUSTROAEGYPTIACUS POUR UTILISATION EN TANT QU'AGENT ANTIULCÉREUX ET/OU AGENT ANTI-HELIOBACTER PYLORI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.12.2014 Bulletin 2014/49**

(73) Proprietor: **King Saud University**
**11421 Riyadh (SA)**

(72) Inventors:
- **Awaad, Amani Shafeek**
  **11333 Riyadh (SA)**
- **Alrifai, Asmaa Abdullah**
  **11333 Riyadh (SA)**
- **El-Desouky Zain, Mohamed**
  **11333 Riyadh (SA)**
- **El-Meligy, Reham Moustafa**
  **11333 Riyadh (SA)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**EP-A1- 1 588 705**

- **Anonymous: "Convolvulus austro-aegyptiacus Abdallah & Sa'ad", Les conservatoire et jardin botaniques de la Ville de Genève -African Plant Database , XP002712186, Retrieved from the Internet: URL:http://www.ville-ge.ch/musinfo/bd/cjb/africa/details.php?langue=an&id=139778 [retrieved on 2013-09-02]**
- **SAIRAM K ET AL: "Effect of Convolvulus pluricaulis Chois on gastric ulceration and secretion in rats", INDIAN JOURNAL OF EXPERIMENTAL BIOLOGY, vol. 39, no. 4, April 2001 (2001-04), pages 350-354, XP009172193, ISSN: 0019-5189**
- **VIMAL S K ET AL: "Anti-bacterial activity of herbal extracts, EuMiL(R) and antibiotics against Helicobacter muridarum", PHARMACOGNOSY JOURNAL, vol. 2, no. 11, 2010, pages 436-441, XP002712187, PHARMACOGNOSY NETWORK WORLDWIDE IND ISSN: 0975-3575**
- **ATTA ATTIA H ET AL: "Phytochemical and pharmacological studies on Convolvulus fatmensis Ktze.", JOURNAL OF NATURAL REMEDIES, vol. 7, no. 1, January 2007 (2007-01), pages 109-119, XP009172192, ISSN: 0972-5547**
- **KAPADIA NAYANA S ET AL: "A pharmacognostical study on Convolvulus prostratus Forssk.", JOURNAL OF NATURAL REMEDIES, vol. 5, no. 2, June 2005 (2005-06), pages 108-114, XP002712188, ISSN: 0972-5547**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SIRIMA MAHATTANADUL ET AL: "Effects ofaqueous fruit extract and its biomarker scopoletin on reflux esophagitis and gastric ulcer in rats", JOURNAL OF ETHNOPHARMACOLOGY,, vol. 134, no. 2, 6 December 2010 (2010-12-06), pages 243-250, XP028162611, ISSN: 0378-8741, DOI: 10.1016/J.JEP.2010.12.004 [retrieved on 2010-12-14]
- AMANI S. AWAAD ET AL: "New Activities for Isolated Compounds from Convolvulus austro-aegyptiacus as Anti-ulcerogenic, Anti-Helicobacter pylori and Their Mimic Synthesis Using Bio-guided Fractionation", PHYTOTHERAPY RESEARCH, 10 June 2015 (2015-06-10), pages n/a-n/a, XP055208228, ISSN: 0951-418X, DOI: 10.1002/ptr.5379

## Description

The present invention relates to an extract and isolated compounds from *Convolvulus austroaegyptiacus* for use in the treatment of ulcers and/or *Helicobacter pylori* infections.

Family *Convolvulaceae* contains a large number of plants where the largest genus is *Convolvulus* which comprises 250 species present as trees, shrubs or herbs.

Most of *Convolvulus* plants contain alkaloids, flavanoids and coumarins. In addition, they possess many pharmacological activities including cytotoxic, antioxidant, anti-inflammatory and antiulcer activities and used in treatment of coughs and asthma.

Peptic ulcer is a common gastrointestinal disorder in modern era. It becomes a common global health problem affecting a large number of people worldwide. There are different classes of drugs that have been used in the treatment of peptic ulcer, most of them exhibits serious side effects like; arrhythmias, gynaecomastia, arthralgia and hypergastrinemia (Awaad et al. Journal of Saudi Chemical Society, 2012, 17, 101-124).

*Helicobacter pylori* is recognized as a major etiologic agent in gastrodudenal diseases including chronic gastritis, peptic ulcers, gastric adenocarcinoma and mucosa associated lymphoid tissue lymphoma (MALToma). The present treatment regimes for *H. pylori* infections are based on the combination of a proton pump inhibitor and two antibiotics (triple therapy).

However, the use of antibiotics leads to increasing development of bacterial resistance which has compromised the efficacy of this treatment. Therefore, the search for new effective therapeutic agents is of paramount importance. In this regard, many species of plant (and their derivates and isolated compounds), extracts of the lichen *Certaria islandica,* Chinese green tea and several native plants used in folk medicine for the relief of gastric symptoms have been studied to establish their pharmacological activity and shown to have *in vitro* antibacterial activity against *H. pylori.*

In fact, number of drugs and natural substances such as various essential oils, extracts of the lichen *Certaria islandica,* Chinese green tea and several native Iranian plants have been shown to have *in vitro* antibacterial activity against *H. pylori.*

Vimal et al., Pharmacognosy Journal, 2010, 2(11), 436-441 discloses anti-bacterial activity of herbal extract and antibiotics against *Helicobacter muridarum.*

Atta Attia et al., Journal of Natural Remedies, 2007, 7(1), 109-119 discloses Phytochemical and Pharmacological Studies on Convolvulus fatmensis Ktze.

Kapadia Nayama et al., 2005, 5(2), 108-114 discloses a Pharmacognostical Study on Convolvulus prostratus Forssk.

Sirima Mahattanadul et al., Journal of Ethnopharmacology, 2010, 134(2), 243-250 is related to effects of aqueous fruit extracts and its biomarker scopoletin on reflux esophagitis and gastric ulcer in rats.

In the discovery of new anti-ulcer drugs, special interest has been directed to the natural products. Traditional medicine has proved to be clinically effective and relatively less toxic than the existing drugs and also reduces the offensive factors serving as a tool in the prevention of peptic ulcer.

Therefore, it is an object of the present invention to provide agents for treatment of ulcer and/or *Helicobacter pylori* which overcome the drawbacks of the prior art, in particular to provide agents being safe and having no significant toxicity, which can be obtained from natural materials, for example plant materials.

This object is achieved in accordance with the subject-matter of the independent claims. Preferred embodiments result from the sub-claims.

The object is particularly achieved by an extract from a plant material of *Convolvulus austroaegyptiacus* for use in the treatment of ulcers and/or *Helicobacter pylori* infections.

Preferably, the extract for use in the treatment of ulcers is an alcoholic extract, preferably is a methanolic extract.

Even preferred, the plant material comprises the aerial parts of *Convolvulus austroaegyptiacus.*

The object is further achieved by scopolin for usein the treatment of ulcers.

Further described herein is a method for isolating scopolin and/or scopoletin from *Convolvulus austroaegyptiacus,* comprising the steps:

a) Extracting a plant material of *Convolvulus austroaegyptiacus* to obtain an extract;

b) concentrating the extract to obtain a concentrate;

c) chromatographing the concentrate into fractions; and

d) isolating fractionated compounds.

The plant material may comprise the aerial parts of *Convolvulus austroaegyptiacus.*

The extracting may be carried out by using at least one organic solvent.

The organic solvent may be selected from the group consisting of petroleum ether, heptane, hexane, ethanol, isopropanol, methanol and mixtures thereof, preferably from the group of alcohols, most preferably is methanol.

Chromatographing may be carried out by means of thin layer chromatography (TLC), column chromatography, gel filtration or mixtures thereof.

The eluent used for chromatographing may be petroleum ether, heptane, ethanol, methanol, benzene, diethyl ether, chloroform, dichloromethane, water or mixtures thereof.

The stationary phase used for chromatographing may be silica gel, aluminium oxide, crossed-linked dextran gel or mixtures thereof.

Furthermore, the object is achieved by a pharmaceutical composition comprising the inventive extract and/or at least one of the inventive compounds for use in the treatment of ulcers.

Finally, the object is achieved by the inventive pharmaceutical composition or the inventive pharmaceutical composition for usein the treatment of ulcers, formulated for oral administration.

It was surprisingly found by the inventors that extracts and/or compounds obtained from a natural material of *Convolvulus austroaegyptiacus* can successfully be used in the treatment of ulcer and/or *Helicobacter pylori* by being not toxic at the same time.

Without limiting the scope of the claims, the present invention can be summarized as follows:
Two coumarins were isolated from *Convolvulus austroaegyptiacus* for the first time and were identified as scopoletin and scopolin. The total alcohol extract (500 and 1000 mg/kg) and the isolated coumarins (50 mg/kg) showed potent anti-ulcer activity in absolute ethanol-induced ulcer in rats. These activities were compared with the standard drug ranitidine (100 mg/kg). Scopolin was the most effective agent in this study, by showing 90.8% protection of control ulcer. These results were well supported by the *in-vitro* anti-*H. pylori* study; scopolin also showed the highest inhibition zones and lowest MICs. The cytoprotective mechanism and the anti-*H. pylori* activity may explain the potent anti-ulcer activity of the total alcohol extract and the isolated coumarins. The acute toxicity study of the extract showed that the extract was highly safe as the LD50 was more than 4000 mg/kg, and these results were well supported by the subchronic toxicity, as the extract administrated to rats for 15 consecutive days at dose 1000 mg/kg showed no alteration in the liver and kidney functions.

"Extracting" in terms of the present invention, for preparing the inventive extract or in the first step of the inventive method, can comprise only one or more than one consecutive extraction steps. In the latter case, different solvents or solvent mixtures, in particular solvents or solvent mixtures having a significantly different polarity can be used in the various steps.

In the same way, "chromatographing" in terms of the present invention can comprise only one chromatographic separation as well as more than one consecutive chromatographic separation steps. Particularly, different eluents and/or stationary phases can be used in each chromatography step. Further, in case that two or more chromatographic separation steps are carried out, each step can be a different chromatographic method, for example in the first step a mixture (concentrate) is separated by column chromatography and single fraction obtained in the first step is subsequently separated by thin layer chromatography.

The term "pharmaceutical composition", as used herein, is intended to comprise at least one pharmaceutically active extract of the present invention and/or at least one of the isolated compound of the present invention and/or corresponding salts thereof.

The pharmaceutical composition can be, for example, in a liquid form, e.g. a solution, sirup, elixir, emulsion and suspension, or in a solid form, e.g. a capsule, caplet, tablet, pill, powder and suppository. Granules, semi-solid forms and gel caps are also considered. In case that the pharmaceutical composition is a liquid or a powder, dosage unit optionally is to be measured, e.g. in the dosage unit of a teaspoon. In addition to one of the extracts or the isolated compounds, the pharmaceutical composition can comprise, for example, flavoring agents, sweeteners, dyes, stabilizers, diluents, suspending agents, granulating agents, lubricants, binders and disintegrating agents. A tablet, for example, can be coated. All of the formulations mentioned can be intended for immediate-release, timed-release and sustained release.

All components of the pharmaceutical composition have to be pharmaceutically acceptable. The term "pharmaceutically acceptable" means at least non-toxic. The therapeutically active compound should preferably be present in the above-mentioned pharmaceutical composition in a concentration of about 0.1 to 99.5% by weight, preferably of about 0.5 to 95% by weight of the total mixture.

The above-mentioned pharmaceutical composition can further contain other pharmaceutical active compounds in addition to the active extracts and/or compounds according to the invention.

Additional features and advantages of the present invention will become apparent in the following detailed description on the basis of the examples.

## Examples

### 1. Preparation of Extracts and Isolation of Compounds

#### a) Plant material

Aerial parts of *Convolvulus austroaegyptiacus* were collected during flowering stage in March 2012 from Riyadh territory. The sample was identified by *Dr. Jacob Thomas*; assistant professor of taxonomy, Botany and Microbiology Department, Faculty of Science, King Saud University, and specimen were kept in the herbarium of Chemistry Department.

Samples of the aerial parts were air dried in shade, reduced to fine powder and kept for phytochemical and biological investigation.

#### b) Extraction

One kilogram air dried powder of the investigated plant Convolvulus austroaegyptiacus was extracted by refluxing in methanol (2 liters) for 3 hours and then filtered off. The plant was re-extracted again for three times by the same way. The combined filtrates were concentrated under reduced pressure at temperature not exceeding 30°C to obtain residue (149 g).

These were each subjected to preparative thin layer chromatography using solvent system (a). Bands corresponding to each flavonoid were separately extracted with methanol, concentrated and for final purification each one was submitted to a column of Sephadex LH-20 eluted with methanol: water 1:1.

For the isolation of the compounds from the alcohol extract, the residue obtained (50 g) was carried out using column chromatography packed with Silica Gel and eluted gradually with ethyl acetate-methanol-water. Two compounds were obtained in semi-purified condition. Further purification was performed by passing each compound through a column of Sephadex LH-20 using methanol-water as eluent, where two compounds were isolated and symbolized as C1&C2.

#### c) Identification of the Isolated Compounds:

The isolated compounds were identified using different physical and spectral methods; melting point, UV and IR spectra, $^{1}$H-NMR, $^{13}$C-NMR, Cosy-NMR and Mass spectrometer as scopoletin and scopolin.

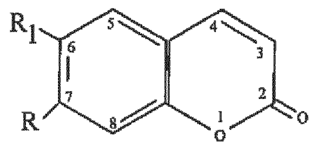

| | **R** | **$R_1$** |
|---|---|---|
| 1. Scopoletin | OH | $OCH_3$ |
| 2. Scopolin | O-glucose | $OCH_3$ |

*Structures of the isolated compounds*

**Compound C1:** (30 mg) white crystals, $R_f$ = 0.33 (system a); mp. 203-204°C. UV, $\lambda_{max}$ (MeOH): (nm) 228, 252, 260, 295, 344; (NaOAc) 245, 277, 391. $^{1}$H-NMR (DMSO-$d_6$): δ 7.9 (1H , J=9, H-4); δ 7.2 and 6.75 (2H, 2S, H-5 and H-8, respectively); δ 6.2 (1H , d, J=9 Hz, H-3) and δ 3.8 (3H, S, $OCH_3$). This compound was identified as **Scopoletin.**

**Compound C2:** (25 mg), white crystals, $R_f$ = 0.45 (system b); mp. 214-216 °C. UV, $\lambda_{max}$ (MeOH): (nm) 276, 347; (NaOAc) 258, 389. $^{1}$H-NMR (DMSO-$d_6$): δ 7.95 (1H, d, J=9 Hz, H-4); δ 6.7 (1H, d, J=8Hz), 5.15 (1H, d, H-1 glucose); sugar protons at 3.33- 3.78. H-5; δ 7.1 (1H, S, H-8); δ 6.31 (1H, d, J=9 Hz, H-3) and δ 3.9 (3H, S, $OCH_3$). $^{13}$C-NMR (DMSO-$d_6$): δ 160.40 (C-2) 112.14 (C-3), 145.88 (C-4), 109.60 (C-5), 113.19 (C-6), 149.80 (C-7), 102.91(C-8), 153.67 (C-9), 112.23 (C-10), 55.90 (O-$CH_3$), 100.00 (C-1'), 75.8 (C-2'), 76.64 (C-3'), 71.70 (C-4'), 77.01 (C-5'),

69.99 (C-6'). This compound was identified as **Scopolin.**

**d) Preparation of the plant extract**

Dried aerial parts of *C. austroaegyptiacus* (100 g) were extracted by percolation in 90% ethanol at room temperature for two days. The ethanol extract was filtered and the residues were re-percolated for four times. The total ethanol extract was concentrated under reduced pressure at a temperature not exceeding 35°C to yield a dry extract of 25 g. The dried plant extract was freshly suspended in distilled water just before administration by the aid of Tween 80.

**2. Biological Activities**

**a) Animals**

Swiss albino mice of both sex (26-30 g) and male Wistar rats (180-200 g) were supplied by the animal house of King Saud University, KSA. Animals were housed in standard polypropylene cages with wire mesh top and maintained under standard conditions (temperature 23±1.0 °C, humidity 55±10%, 12 h light/12 h dark cycle). They were fed with a standard pellet diet with water *ad libitum* and were allowed to adapt to the laboratory environment for one week before experimentation.

**b) Preliminary Phytochemical screening:**

For determination of the active constituents, the air dried powders of the investigated plant *C. austroaegyptiacus* were subjected to preliminary phytochemical screening according to the published methods (Ayoola et al. Tropical Journal of Pharmaceutical Research, 2008, 7 (3), 1019-1024).

**c) Determination of median lethal dose ($LD_{50}$)**

The oral median lethal dose ($LD_{50}$) of the alcohol extract of *C. austroaegyptiacus* was determined as described by Lorke Arch. Toxicology, 1983, 54, 275-287. Swiss albino mice in groups of six, received one of 500, 1000, 2000, or 4000 mg/kg doses of the tested extract. Control animals were received the vehicle and kept under the same conditions. Signs of acute toxicity and number of deaths per dose within 24 h were recorded.

**d) Anti-ulcero genie activity**

Evaluation of the anti-ulcerogenic activity was carried out using absolute ethanol-induced ulcer model as described by Bighettia et al. Phytomedicine, 2005, 12, 72-77.

About 36 Wistar rats were used, they divided into 6 groups each of 6 rats. Group 1 received the vehicle and served as control group, group 2 received ranitidine (100 mg/kg) and served as standard group, groups 3 and 4 received the total alcohol extract of the plant under investigation at doses 500 and 1000 mg/kg groups from 5 and 6 received the isolated compounds scopoletin and scopolin at dose 50 mg/kg respectively.

Rats of all groups were fasted for 24 h then all medications were administered orally. One hour after treatment, the animals received an oral dose of absolute ethanol (1 ml/200 g) and then sacrificed one hour later, by ether inhalation, the stomachs were rapidly removed, opened along their greater curvature and gently rinsed under running tap water.

***Number of lesions*** in the glandular part of the stomach were measured under an illuminated magnifying microscope (10 x). Long lesions were counted and their lengths were measured. Petechial lesions were counted, and then each five petechial lesions were taken as 1 mm of ulcer.

***The lesion scores:*** The mucosal lesions were quantified by the scoring system (0-5) 0 = no damage; 1 = Local edema and inflammation without ulcers; 2 = One ulcer without inflammation; 3 = one to two ulcers with inflammation & lesion diameter < 1 cm; 4 = More than two ulcers with lesion diameter 1-2 cm; 5 = Sever ulceration with lesion diameter >2 cm.

***Ulcer index:*** To calculate the ulcer index (mm), the sum of the total length of long ulcers and petechial lesions in each group of rats was divided by its number. The curative ratio was determined according to the formula:

$$\%\ \text{Protection of control ulcer} = \text{Control UI} - \text{Test UI} / \text{Control UI} \times 100$$

The present results showed that the plant extract at doses of 500 and 1000 mg/kg possessed a potant anti-ulcerogenic activity against ulcer-induced by absolute alcohol. It produced a percent protection of control ulcer by 61.7%

and 80% respectively which are more effective than ranitidine which produce 33.3%. The isolated compounds scopoletin and scopolin (50 mg/kg), showed significant anti-ulcerogenic activity. Scopolin was the most effective compound in the present study, it produced percent protection of control ulcer by 90.8% and it was mainly responsible for the activity of the investigated plant (Table 1).

**Table 1: Antiulcerogenic effect of extract and isolated compounds from *Convolvulus austroaegyptiacus.***

| Groups | Dose mg/kg | score | no of ulcers | ulcer index | % protection |
|---|---|---|---|---|---|
| **Control** | - | 4 | 15.0±1.24 | 12.0±1.74 | 0 |
| **Ranitidine** | **100** | 2.2 | 7.6**±1.05 | 8.0*±1.24 | 33.3 |
| **Total alcohol extract** | **500** | 2 | 4.8***±0.25 | 4.6**±1.78 | 61.7 |
| **Total alcohol extract** | **1000** | 1.25 | 1.0***±0.3 | 2.4***±0.49 | 80 |
| **Scopoletin** | **50** | 1.5 | 5.8***±1.49 | 5.1***±1.67 | 57.5 |
| **Scopolin** | **50** | 1 | 1.2***±0.17 | 1.1***±0.19 | 90.8 |
| Data are expressed as mean ± SD, n=6, * $p \leq 0.05$, ** $p \leq 0.01$, *** $p \leq 0.001$ | | | | | |

**e) *In-vitro Anti-Helicobacter pylori***

**i) Bacterial isolates**

A total of seven clinical isolates of *Helicobacter pylori* were isolated from 19 biopsies received from patients diagnosed with gastritis or peptic ulcer disease at Al-Kasr Al-Ainy hospital, Cairo, Egypt. Clinical isolates were symbolized from KA1 to KA7. Isolates were grown in Brucella agar plates (Difco, Detroit, Michigan, USA) containing 10% v/v sheep serum at 37°C. Identification was carried out using Gram stain and catalase, oxidase and urea hydrolysis activities. *H. pylori* ATCC 43504 was used as control.

**ii) Disk diffusion test**

The disk diffusion test was used as screening to determine the susceptibility of *H. pylori* isolates and reference strain ATCC 43504 to plant extracts. The bacterial suspensions were spread-plated onto Brucella Agar plates (Difco, Detroit, USA) supplemented with 10% defibrinated sheep blood. Filter paper disks of 6 mm diameter impregnated with 5 mg of each extract (50 μl of stock solutions) were placed onto the surface of the inoculated agar. The plates were incubated at 37°C under microaerophilic conditions and observed after 2 to 5 days.

**iii) Determination of the minimum inhibitory concentration (MIC)**

Minimum inhibitory concentration (MIC) was carried out by the broth microdilution assay (Hachem et al. Diagnostic Microbiology Infections Disease, 1996, 24, 37-41). A total of 100 μL of BHI broth supplemented with 10% defibrinated sheep blood inoculated with $6\times10^8$ *Helicobacter pylori* (McFarland turbidity standard 2) and 100 μL of serial dilutions of *HECb,* and *BI* dissolved in 2% Tween (v/v) was added to each well in the microplate, to reach final concentrations of 25; 50; 100; 200; 400 and 800 μg/mL. The standard drug, Clarithromycin, was diluted to the same concentrations. The microplate was incubated at 37°C under microaerophilic conditions in an atmosphere of 5-15% $O_2$ and 5-10% $CO_2$, for 48-72 h. After incubation, the plates were visually examined, the optical density was determinates at 450 nm and each well was replicated in blood agar (Mueller-Hinton agar with 5% sheep blood), to determine the MIC.

**iv) Determination of median Lethal Dose ($LD_{50}$)**

The total alcohol extract *C. austroaegyptiacus* did not produce any behavioral changes or mortality in treated mice in doses up to 4000 mg/kg. Therefore, the tested plant can be categorized as highly safe since substances possessing $LD_{50}$ higher than 50 mg/kg are non-toxic (Soliman et al., Pharmaceutical Biology, 2012, 50(1), 105-112).

***v) In-vitro* Anti-*Helicobacter pylori***

The activity of total alcohol extract, scopoletin and scopolin; compounds isolated from *Convolvulus austroaegyptiacus,* against *Helicobacter pylori* was determined (Table 3). Seven isolates of *H. pylori* were obtained from 15 gastric biopsies and their susceptibility to total alcohol extract, two antibiotics and compounds isolated from *Convolvulus austroaegyptiacus* was determined by disc diffusion (Table 4). All the isolates of *H. pylori,* with the exception of isolate KA01 that was resistant to Amoxicillin, were sensitive to total alcohol extract, scopoletin, scopolin, amoxicillin and erythromycin.

The results revealed that the highest inhibition zones, 18 and 14 mm, were obtained by scopolin against *H. pylori* ATCC 43504 and KA05, respectively. On the other hand, the lowest inhibition zones 7&8 mm were obtained by scopoletin against *H. pylori* KA02, and KA03, scopolin against *H. pylori* KA03 (Table 3).

**Table 3. Activity of plant extracts against clinical isolates *of H. pylori.***

| ***H. pylori* strains** | **Inhibition zone (mm)** | | | | |
|---|---|---|---|---|---|
| | **Total alcohol extract (5 mg)** | **Scopoletin (5 mg)** | **Scopolin (5 mg)** | **Amoxicillin (30 μg)** | **Erythromycin (15 μg)** |
| KA01 | 10 | 8 | 10 | R | 19 |
| KA02 | 10 | 7 | 14 | 20 | 23 |
| KA03 | 9 | 7 | 8 | 21 | 22 |
| KA04 | 10 | 9 | 11 | 20 | 22 |
| KA05 | 12 | 11 | 12 | 22 | 20 |
| KA06 | 10 | 8 | 11 | 23 | 24 |
| KA07 | 11 | 8 | 13 | 25 | 23 |
| ATCC 43504 | 10 | 7 | 18 | 30 | 2 8 |

The minimum inhibitory concentration (MIC) was determined for the two compounds; total alcohol extract, scopoletin and scopolin, isolated from *Convolvulus austroaegyptiacus* using broth dilution method (Table 2). The results showed that the lowest minimum inhibitory concentration (50 μg/mL) was obtained by scopoletin against *H. pylori* KA05 and by scopolin against *H. pylori* KA04, KA05, KA06, ATCC 43504 and KA07 (50, 45, 60, 45 μg/mg respectively) (Table 4).

**Table 4. Minimum inhibitory concentration (MIC) of plant extracts against clinical isolates *of H. pylori.***

| ***H. pylori* strains** | **Concentration** | | |
|---|---|---|---|
| | **Alcohol extract (μg/mL)** | **Scopoletin (μg/mL)** | **Scopolin (μg/mL)** |
| KA01 | 200 | 100 | 1 00 |
| KA02 | 200 | 100 | 100 |
| KA03 | 100 | 100 | 100 |
| KA04 | 100 | 100 | 50 |
| KA05 | 100 | 50 | 50 |
| KA06 | 200 | 200 | 50 |
| KA07 | 200 | 200 | 50 |
| ATCC 43504 | 200 | 200 | 100 |
| **f) Effect on Liver and Kidney Functions:** | | | |

Male Wister rats were divided into 2 equal groups each of 10 rats. The 1$^{st}$ group was left as a control and administrated water orally, while the 2$^{nd}$ group was orally given the plant extracts in a dose of 1000 mg/kg for 15 days. Blood samples were collected from the orbital plexus of rats, 6 hr after the last dose. Samples were left to clot at room

temperature for 30 min then centrifuged at 1000 rpm for 20 min.

The collected sera were used for determination of the activity of both (AST) aspirate aminotransferase and (ALT) alanine aminotransferase as a liver markers. In addition, levels of blood urea, serum creatinine were also estimated as a kidney markers (Awaad et al. Phytother. Res. 2013, 27, 126-130).

Both liver and kidney functions were not affected before and after treatments as there is no significant difference between control and test group in all experiments, at the 0.05 level of probability (Table 2).

**Table 2: Effect of *C. austroaegyptiacus* extract on liver and kidney functions of rats.**

| Groups | ALT(U/l) | AST(U/l) | Blood Urea (mg/dl) | Serum Creatinine (mg/dl) |
|---|---|---|---|---|
| **Control** | 4.8±0.37 | 5.2±0.37 | 45.50±1.36 | 0.82±0.02 |
| ***C. austroaegyptiacus* extract (1000 mg/kg)** | 5.1±0.22 | 5.3±0.39 | 46.00±1.9 | 0.84±0.02 |
| Data are expressed as mean ± SD, n=10 | | | | |

These results showed that, the alcohol extract of the investigated plant did not reveal hepatotoxic manifestation. In addition, no apparent nephrotoxic manifestations were recorded; this indicated that no side effects were obtained the alcohol extract of *C. austroaegyptiacus.*

The features disclosed in the foregoing description and in the claims may, both separately or in any combination, be material for realizing the invention in diverse forms thereof.

## Claims

1. Extract from a plant material of *Convolvulus austroaegyptiacus* for use in the treatment of ulcers and/or *Helicobacter pylori* infections.

2. Extract for use in the treatment of ulcers and/or *Helicobacter pylori* infections according to claim 1, wherein the extract is an alcoholic extract, preferably is a methanolic extract.

3. Extract for use in the treatment of ulcers and/or *Helicobacter pylori* infections according to claim 1 or 2, wherein the plant material comprises the aerial parts of *Convolvulus austroaegyptiacus.*

4. Scopolin for use in the treatment of ulcers and/or *Helicobacter pylori* infections.

5. Pharmaceutical composition comprising an extract according to any of the claims 1 to 3 and/or the compound according to claim 4 for use in the treatment of ulcers and/or *Helicobacter pylori* infections.

6. Pharmaceutical composition according to claim 5, for use in the treatment of ulcers and/or *Helicobacter pylori* infections, formulated for oral administration.

## Patentansprüche

1. Extrakt aus einem Pflanzenmaterial des *Convolvulus austroaegyptiacus* zur Verwendung bei der Behandlung von Geschwüren und/oder *Helicobacter pylori* Infektionen.

2. Extrakt zur Verwendung bei der Behandlung von Geschwüren und/oder *Helicobacter pylori* Infektionen nach Anspruch 1, wobei das Extrakt ein alkoholisches Extrakt, vorzugsweise ein methanolisches Extrakt, ist.

3. Extrakt zu Verwendung bei der Behandlung von Geschwüren und/oder *Helicobacter pylori* Infektionen nach Anspruch 1 oder 2, wobei das Pflanzenmaterial die oberirdischen Teile des *Convolvulus austroaegyptiacus* enthält.

4. Scopolin zur Verwendung bei der Behandlung von Geschwüren und/oder *Helicobacter pylori* Infektionen.

5. Pharmazeutische Zusammensetzung, umfassend ein Extrakt nach einem der Ansprüche 1 bis 3 und/oder die Verbindung nach Anspruch 4 zur Verwendung bei der Behandlung von Geschwüren und/oder *Helicobacter pylori* Infektionen.

6. Pharmazeutische Zusammensetzung nach Anspruch 5, zur Verwendung bei der Behandlung von Geschwüren und/oder *Helicobacter pylori* Infektionen, hergerichtet zur oralen Gabe.

## Revendications

1. Extrait d'une matière végétale de *convolvulus austroaegyptiacus* destiné à l'utilisation dans le traitement d'ulcères et/ou d'infections par *helicobacterpylori.*

2. Extrait destiné à l'utilisation dans le traitement d'ulcères et/ou d'infections par *helicobacter pylori* selon la revendication 1, dans lequel l'extrait est un extrait alcoolique, de préférence un extrait méthanolique.

3. Extrait destiné à l'utilisation dans le traitement d'ulcères et/ou d'infections par *helicobacter pylori* selon la revendication 1 ou 2, dans lequel la matière végétale comprend les parties aériennes du *convolvulus austroaegyptiacus.*

4. Scopoline destinée à l'utilisation dans le traitement d'ulcères et/ou d'infections par *helicobacter pylori.*

5. Composition pharmaceutique comprenant un extrait selon l'une quelconque des revendications 1 à 3 et/ou composé selon la revendication 4 destiné à l'utilisation dans le traitement d'ulcères et/ou d'infections par *helicobacter pylori.*

6. Composition pharmaceutique selon la revendication 5 destinée à l'utilisation dans le traitement d'ulcères et/ou d'infections par *helicobacter pylori,* formulée pour l'administration orale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **AWAAD et al.** *Journal of Saudi Chemical Society,* 2012, vol. 17, 101-124
- **VIMAL et al.** *Pharmacognosy Journal,* 2010, vol. 2 (11), 436-441
- **ATTA ATTIA et al.** *Journal of Natural Remedies,* 2007, vol. 7 (1), 109-119
- **KAPADIA NAYAMA et al.** *Pharmacognostical Study on Convolvulus prostratus Forssk,* 2005, vol. 5 (2), 108-114
- **SIRIMA MAHATTANADUL et al.** *Journal of Ethnopharmacology,* 2010, vol. 134 (2), 243-250
- **AYOOLA et al.** *Tropical Journal of Pharmaceutical Research,* 2008, vol. 7 (3), 1019-1024
- *Lorke Arch. Toxicology,* 1983, vol. 54, 275-287
- **BIGHETTIA et al.** *Phytomedicine,* 2005, vol. 12, 72-77
- **HACHEM et al.** *Diagnostic Microbiology Infections Disease,* 1996, vol. 24, 37-41
- **SOLIMAN et al.** *Pharmaceutical Biology,* 2012, vol. 50 (1), 105-112
- **AWAAD et al.** *Phytother. Res.,* 2013, vol. 27, 126-130